Europäisches Patentamt

European Patent Office

Office européen des brevets

(11) Publication number: 0 349 851
A2

# EUROPEAN PATENT APPLICATION

(21) Application number: 89111456.3

(22) Date of filing: 23.06.89

(51) Int. Cl.4: C12N 5/16 , C12P 21/08 , C12P 21/00

(30) Priority: 04.07.88 IT 2122288

(43) Date of publication of application:
10.01.90 Bulletin 90/02

(84) Designated Contracting States:
AT BE CH DE ES FR GB GR IT LI LU NL SE

(71) Applicant: GENETIK MAB SRL
Baluardo Partignani, 13
I-28100 Novara(IT)

(72) Inventor: Ghielmi, Salvatore
Via Ambaraga, 22
I-25100 Brescia(IT)

(74) Representative: Petraz, Gilberto Luigi
G.L.P. S.a.s. di Gilberto Petraz P.le Cavedalis
6/2
I-33100 Udine(IT)

(54) N-oma cell lines as means for immortalizing substance producing cells of any animal species.

(57) The present invention relates to the immortalization, by somatic genetic manipolation of any substance producing cells, without dampering in anyway the physiological characteristics of said cells. An immortalized cell is fused to a substance producing cell and the resulting hybridoma is suitably selected and refused for at least two further consecutive times to the substance producing cells. At each fusion step, the relative content of the genome of the two fusion partners is determined and those hybrid cells containing the last immortalized cell genome are selected as partner for subsequent fusions. After "n" cycles of fusions the resulting cells, here defined as n-omas, will have retained only those genes of the immortalized cell necessary to assure the immortalized phenotype. These cells, however, will contain the entire genome of the substance producing cell. This method can be applied to immortalize any substance producing cell and especially cells from animal species in which suitable fusion partners are not available.

## N-OMA CELL LINES AS MEANS FOR IMMORTALIZING SUBSTANCE PRODUCING CELLS OF ANY ANIMAL SPECIES.

The present invention relates to a process for the preparation of in so far called n-oma cell lines suitable as fusion partner with substance producing cells, comprising the step of fusing an immortalized cell fused to a non-transformed cell with a non-transformed cell of the same or different origin for at least two further consecutive times.

The present invention relates as well to a continuous, fast growing, non-secreting n-oma cell lines, clones and sub-clones thereof, which are the fusion product between a substance producing cell and a hybrid cell, which hybrid cell is the fusion product between a substance producing cell and an immortalized cell.

The present invention relates also to a continuous, stable, secreting n-oma cell line which is the fusion product between the n-oma cell line described above and a substance producing cell.

The present invention relates also to the above described n-oma cell lines which allow the production of monoclonal antibodies in any desired animal species.

These lines are obtained by fusing an immortalized cell of one species to substance producing cells of an animal of another species.

These "first generation hybridomas" are usually genetically unstable and tend to loose chromosomes of the non-transformed parental cell.

The invention consists in fusing these "first generation hybridomas" to activated substance producing cells of the same species used for the first fusion.

The fusion can be repeated "n" times, where "n" is 3, 4, 5 or more.

Thus, if M is the original transformed cell, if S is the substance producing cell of the desired species, and if "n" is the number of fusions, the cycle could be represented as
$$(M \times S) \times S^{n-1}$$

We call the resulting cell lines n-omas.

This invention consists in consecutively introducing genetic material of the desired animal species into the original transformed cell.

After some cycles this results into the loss of genetic material of the immortalized cell and in the selective retention of the chromosomes of the non-transformed cell fusion partner.

Since the normal cells fused are substance producing cells and since the selection is partly based on the retention of the transformed phenotype, the resulting n-oma will have the property to continuously divide and to stably produce the substance of the desired species.

The invention can be used to obtain large quantities of the desired substance i.e. hormons, enzymes, factors, antigens, toxins or any genetically modified cells able to express foreign proteins.

Antibodies, for instance, are complex proteins produced by B lymphocytes and they play a fundamental role in the defence of the organism against pathogens.

They are produced after active or passive immunization with a given antigen.

Due to the enormous diversity of combining sites of antibodies the humoral immune response against a given antigen is generally heterogeneous and consists of a mixture of antibodies, each one produced by a single cell, which recognize different epitopes of the same antigen with various degrees of affinity.

So far, sera obtained from animals immunized with a given antigen contain polyclonal antibodies.

Polyclonal antibodies have a limited practical use because their availability is limited and the isolation of the desired specificity is technically coumbersome.

The original work of Khöler and Milstein (Nature 256, 495-497.1975) has allwed to bypass this problem by describing a methodology to obtain virtually unlimited quantities of antibodies, all derived from a single precursor cell and therefore called monoclonals.

Khöler and Milstein demonstrated that by fusing a myeloma cell with activated B lymphocytes one can obtain hybrid cells that produce a specific antibody.

This procedure has been worldwide successfully used for the obtention of RODENTS (mice and rats) hybridomas and monoclonal antibodies.

Although several authors have described the production of hybridomas in humans and in rabbits, these hybridomas have later been shown to be very unstable and therefore of little practical use.

It is clear that stable and easy production of monoclonal antibodies in species other than rodents is desirable for several reasons: their therapeutic value in humans (for human monoclonals), the high stability of rabbit antibodies compared to that of rodent antibodies.

It is worthy of note that the antibody repertoire recognizes a limited number of epitopes which differ from species to species.

Therefore the availability of monoclonal antibodies rised agaist the same antigen but in different species will provide a panel of reagents which can recognize all the determinants (some of which can be very important for the functional properties of the antigen) expressed by a given antigen.

The number of foreign determinants recognized by the immune system of a given species is inversely related to evolutionary distance between the species from which the immunizing antigen has been derived and the host species.

For example, a human antigen will elicit a more diverse response in birds than in mammalians.

This is particularly relevant in the case of tumor-associated antigens, some of which tend to be conserved in evolution.

In order to obtain human monoclonal antibodies, several groups have tried to directly fuse myeloma cell lines with human B lymphocytes.

Although such fusions were first described ten years ago and have been successful in few cases, these experimental approaches have been limited by the instability of the human chromosomes (SCHWABER and COHEN, Nature 244:444-447, 1973; SCHLOM et al PNAS USA 77:6841-6845, 1980).

In rabbits the situation is even more complex than in humans, since no rabbit myeloma parent cell lines are available which can be used as fusion partners for B cells.

Thus, all attempts to immortalize rabbit B cells must rely either on etherofusions or on artificial immortalisation with retroviral vectors.

A.D. Strosbury et al (PNAS, 71, 263-65, 1974) and J.J. COLLINS et al (PNAS, 71, 260-63, 1974) reported the preparation of a stable antibody-producing but antibody non-secreting rabbit cell line (TRSC-1) obtained upon transformation of spleen cells with Simian virus 40 (SV40).

In 1981, Petit-Koskas et al (Eur. J. Immunol. 11, 338-92, 1981) reported that 0,02% ethymethyl sulfonate (EMSF) causes the mutation of the TRSC-1 line.

The mutated TRSC-1 line, after selection in 6-thioguanine-containing medium, was fused with lymph-node cells from immunized rabbits.

The fusion generated a hybridoma that failed to produce active antibodies.

The present invention can circumvent all problems of chromosomal instability and provide the technique to obtain hybridomas producing large amounts of antibodies in any animal species.

It should be noted that hybridomas of II generations have already been constructed (see GB-A-2,113,715 and GB-A-2,175,918).

The present invention differs from said prior art since the genetic material of the substance producing cells is reintroduced into the immortalized cell n times (where n can be 3, 4, 5 etc.).

Experiments conduced by the inventor have clearly demonstrated that when n = 2 the resulting heterohybrid is highly instable due to the retention of the genetic material of the immortalized cell.

The present invention, however describes a method to obtain n-omas in which the only genetic material of the immortalized cell remaining in said n-omas is the one that confers the immortalizad phenotype while majority of the remaining genoma derives from the substance producing cell.

This genotype can only be achieved when n≥3.

"n" depends on the cell type and on the substance desired and in some cases can be > 5.

A novel method and cell lines are provided for the efficient production of substances produced by cells of various animal species.

An immortalized myeloma cell line, for instance, is fused with lymphoid cells from immunized animals.

The cells are grown in the presence of 6-thioguanine in order to select HAT sensitive hybrids.

The resulting hybridomas are re-fused with lymphocytes from the same animal species and the double-hybrids reselected as before.

This cycle of re-hybridisation with lymphocytes from the species from which the original non-transformed cell is derived, has to be repeated "n" times before the obtention of a HAT sensitive line that can be used as the fusion partner in fusions with lymphocytes of the original species to obtain n-omas that stably produce antibodies.

The originality of the invention consists into selectively introducing by somatic genetic the immortalizing genetic material of one cell into a substance producing cell and thereby transferring the immortalized phenotype to the former cell while mantaining its phenotypic stability.

The present invention can be used to produce large amounts of the desired substances i.e. hormons, enzymes factors, toxins or the products of genetically modified cells.

Another application of the present invention is the production of monoclonal antibodies of the desired animal specie.

For this particular application the first step is the immunization of the animal.

The possibility to use any desired animal allows the use of an illimited number of antigens i.e. tissue antigens (for instance major histocompatibility genes products), bacterial antigens, viral antigens (for instance gp120, gp41 and p24 of HIV-1 virus), parasites and fungi antigens, hormons, enzymes, toxins, etc..

The protocol of immunization varies according to the antigen to be used and to the species whose monoclonals are to be obtained.

The experimental protocols will be the ones already described in the literature that allows the best B cells activation.

As a general rule however the animals will be reimmunized three days before the lymphoid organs are removed for the fusion.

A HAT sensitive non-Ig (immunoglobulins) producing myeloma line is then fused with the animal lymphocytes.

Hybrid cells are selected in HAT medium over a long period of time.

This step is necessary to select those hybridomas which stably retain at least the animal X chromosome which confers the HAT resistant phenotype.

After 40 days hybridomas which do not produce either light chains or heavy chains nor whole antibody molecules are selected and then cultured in the presence of 6-thioguanine to isolate HAT sensitive clones.

Several of these clones are then re-fused to lymphocytes of a second animal previously immunized as the first one.

This second generation of hybridomas are again tested for production of Ig or their single components and negative clones are isolated.

One selection system has been described, as representative of the technique and principles which are generally operative.

Other selection techniques, bnased on other forms of genetic modifications or biochemical inhibition may be employed, as will be readily apparent to those skilled in the art.

This procedure helds a large number of clones which can potentially be used as progenitors of a suitable fusion partner.

It is therefore necessary at this step to select those clones which appear to retain the largest amounts of the animal DNA and the lowest amount of myeloma DNA.

This can be done by quantifying the two species DNA in a cyto-dot assay, where serial dilutions of DNA from the different clones are hybridized to $^{32}P$ labelled ECO-R1 digested liver DNA of the two species.

Those clones which retain the largest amount of animal DNA are again fused with lymphocytes of a third animal immunized as the first two.

The selection and screening procedure will be repeated as described above, according to the formula $(MxS)xS^{n-1}$, where "n" can be any number >3.

The number of cycles necessary to obtain the best potential fusion partner will be determined by the level of animal DNA enrichment after each fusion step.

The selected n-omas will finally be fused to lymphocytes from immunized animals and the hybrid cells obtained will now be screened for the production of antibodies specific for the original antigen and the positive cells will be cloned by limiting diluitions.

Once the desired hybrid has been selected and cloned, the resultant antibody may be produced by in vitro colturing the described n-oma in a suitable medium for a suitable length of time.

These parameters are known or can be easily determined.

Alternatively, it should be possible to inject hybrid cells into irradiated or nude mice or other animals which have been previously treated with pristane.

This will cause the production of the desired antibody in the blood stream and peritoneal exudate of the host after a suitable length of time.

Although the above description relates specifically to antibodies producing cells, the same method can be used for the isolation of any substance producing cells.

The selection of the hybrids and the detection of the substance will be those best suitable for the characteristics of the desired n-omas.

EXAMPLE 1

ESTABLISHMENT OF SGH-n3 RABBIT n-OMA LINE.

A myeloma line designated SP2 described by SHULMAN et al (Nature 276, 265, 1978) was chosen for the first fusion.

The SP2 cell line is characterized as a nonproducer of antibodies and is 6-azaguanine resistant due to defective HPRT enzymatic activity.

Spleen cells of rabbits previously immunized with a monoclonal mouse IgM protein (PTL-3) were fused with SP2.

The fusion medium contained polyethilen-glycol (PEG) 1000 MW at 45% volume in serum-free DMEM.

The SP2 cells and splenocytes were mixed at a ratio of 3 splenocytes per one SP2 cell.

The fusion mixture (containing PEG) was then incubated for 1′ at 37°C and 10 ml of serum-free DMEM medium were added during a 10′ time.

The cells were washed once with DMEM containing 10% heat-inactivated horse serum.

The cell suspension was then aliquoted in 96 well Costar culture microplates in 0.2 ml of culture medium.

The cells were incubated at 37°C in humidified atmosphere containing 10% $CO_2$.

After one day the coltures were fed with 20 ul of 10x HAT medium.

The HAT medium selects for hybrid cells since the SP2 cells are HAT sensitive whereas splenocytes will die after few days in colture.

The supernatants of growing hybrid cells were tested for production of rabbit antibodies in a sandwich-RIA assay using goat anti-rabbit antibodies in solid phase, the supernatants, and $^{125}$I labelled goat-anti-rabbit antibodies.

Ig non-producing hybridomas were selected and expanded in normal medium.

Those hybridomas which grow better were thereafter cultured in the presence of 6 ug/ml of 6-thioguanine.

After few weeks cells that survived to 6-thioguanine were expanded and tested for their sensitivity to HAT medium.

One of the 6-thioguanine resistant clones, called SGH-1, was selected as the partner for a fusion with splenocytes of a rabbit immunized with PTL-3.

Again hybridomas were detected by growth in selective medium and 20 of them were further selected in 6-thioguanine.

12 hybridomas gave rise to 6-thioguanine resistant lines.

At this time it was necessary to select among the 12 hybrids those who display the best characteristics to be used as a partner for further fusion.

To this end, the relative amount of rabbit and mouse DNA was quantified in all these lines (as described in example 3).

One of the hybridomas, called SGH-n2 contained the highest amount of rabbit DNA and the lowest amount of mouse DNA as compared to the others.

This hybrid was therefore used as a partner for a subsequent fusion with splenocytes from rabbits immunized with PTL-3.

Again non-producer hybridomas were detected and selected in 6-thioguanine as described above.

Quantification of mouse and rabbit DNA of the 6-thioguanine resistant clones revealed that in one of these hybrids (SGH-n3) the ratio of rabbit/mouse DNA was close to 2:1.

This n-oma was therefore used as the partner of fusion with immunized rabbit splenocytes to obtain rabbit monoclonal antibodies.

The SGH-n3 line cell has been deposited by: ECACC, PHLS CAMR, Porton Down, Salisbury, Wilthshire SP4 OJG, Great Britain under the filing number 88063001.

EXAMPLE 2

METHOD OF IMMUNIZING RABBITS.

Purified PTL-3 mouse monoclonal antibodies were polymerized in a glass tube in the presence of 0.25% glutheraldeide.

The polymer was diluted in PBS pH 7.4 at the concentration of 0.5 mg/ml.

Then, 0.7 ml of solution was emulsified in an equal volume of complete Freund adjuvant and the emulsion was injected intradermically in the rabbits.

One month later the rabbits received an injection of the same antigen emulsified in incomplete Freund adjuvant.

Three weeks later and three days before fusion the rabbits were again injected intradermically with the antigen in incomplete Freund adjuvant.

EXAMPLE 3

FUSION OF SGH-n3 CELLS WITH SPLENOCYTES FROM IMMUNIZED RABBITS.

A cell suspension of splenocytes from immunized rabbits was obtained by mechanically disrupting the spleen with forceps.

The SGH-n3 cells and splenocytes were then mixed at a ratio of 4 splenocytes per one SGH-n3 cell.

Fusion was performed as described above (example 1) and supernatants from growing hybrids were tested after three weeks.

EXAMPLE 4

SCREENING OF N-OMAS.

The supernatants from the n-omas were first assayed for the presence of rabbit immunoglobulins.

Since the rabbits utilized in this fusion were all of a3a3 b4b4 allotypes we used for the first screening rabbit anti b4 antibodies produced in a a3a3 b6b6 homozigous rabbit.

The test consisted in coating 96 wells polystirene microplates with 1 ug of anti-b4 antibodies for two hours at room temperature.

Thereafter the plates were washed and each well was saturated with 200 ul of PBS containing 0.5% bovine serum albumin (BSA).

After saturation, 100 ul of n-omas supernatant was added to the wells for three hours at 37°C.

After washing each well received received 100 ul of a solution containing $^{125}$I labelled rabbit anti-b4 antibodies (c/a 40,000 cpm).

After two hours incubation at 37°C the plates were washed and bound radioactive material was detected in a gamma-counter.

In a typical experiment we found that supernatants of 70 out 90 n-omas contained b4 positive antibodies.

The second screening consisted in detecting anti-PTL-3 specific antibodies.

For this purpose antibodies-positive supernatants were added to 96 wells polystirene microplates precoated with 4 ug/ml of PTL-3 protein (purified by affinity chromatography) - and saturated with 0.5% BSA in PBS.

After washing, bound anti PTL-3 antibodies were revealed either with $^{125}$I labelled anti b4 antibodies, or with $^{125}$I labelled PTL-3 protein.

5 of 70 antibodies positive supernatants contained anti PTL-3 antibodies.

The 5 positive n-omas were cloned by limiting diluition on $3 \times 10^5$ ml mouse thymocytes.

10 clones of each n-oma were retested for anti-PTL-3 activity and two that produced particularly high levels of specific antibodies were selected and expanded for further analysis.

The monoclonality of these clones was verified by immunoprecipitating the supernatants of the two hybridomas pulsed with 3-H leucine for two hours.

The immunoprecipitated material was then analysed by SDS-PAGE in 10% polyacrilamide gel under reducing conditions.

The presence of two single 25 and 55 Kd bands in the gel observed under reducing conditions demonstrated the monoclonality of the secreted antibodies.

On the basis of radioimmunoassay analysis we estimate that these n-omas produce in culture between 20 and 50 ug/ml of specific antibodies.

These values are much higher than those observed with classical hybridomas.


Claims

1. A process for the preparation of n-oma cell lines suitable as fusion partner with substance producing cells, comprising the step of fusing an immortalized cell to a non-transformed partner and repeating the fusion of the obtained transformed hybrid cell with non-transformed partners of the same or different origin for at least two further consecutive times.

2. A process according to claim 1 wherein the non-transformed partner is a lymphocyte.

3. A process according to anyone of claims 1 and 2 wherein the immortalized cell is selected from myeloma or any immortalized cell.

4. A process according to anyone of claims 1 to 3 wherein the obtained hybrid cells are immortalized cells which do not produce antibodies or a predetermined substance.

5. A process according to anyone of claims 1 to 3 wherein the immortalized cell is of any animal specie origin.

6. A process according to anyone of claims 1 to 5 wherein the transformed hybrid cells are xenogeneic n-omas.

7. A process according to anyone of claims 1 to 6 wherein the fusion takes place in the presence of any fusion promoting substance.

8. A process according to anyone of claims 1 to 7 wherein the selection of the desired n-omas takes place by any genetic modification or biochemical inhibition and by testing for the ability to produce antibodies or a predetermined substance.

9. A process according to claim 8 wherein the selection is achieved by selecting HAT resistent or 6-thioguanine resistent cells.

10. Continuous, fast growing, non secreting n-oma cell lines, clones and subclones thereof which are the fusion product between a substance-producing cell and a hybrid cell, which hybrid cell is the fusion-product between a substance producing cell and a hybrid cell, which hybrid cell is the fusion product between a substance producing cell and an immortalized cell.

11. Continuous n-oma cell lines according to claim 10 which are the fusion product of at least three fusion steps.

12. Continuous n-oma cell lines according to anyone of claims 10 and 11 wherein the substance producing cell is a lymphocyte.

13. Continuous n-oma cell lines according to anyone of claims 10 to 12 wherein the immortalized cell is selected from myelomas or any immortalized cell.

14. Continuous n-oma cell lines according to anyone of claims 10 to 13 wherein said cells are HAT sensitive.

15. Continuous n-oma cell line according to anyone of claims 10 to 14 which is a xenogeneic n-oma which does not secrete antibodies or a predetermined substance.

16. A process for producing a predetermined substance comprising the step of fusing the continuous n-oma cell line according to anyone of claims 10 to 15 with a substance producing cell.

17. A process accoding to claim 16 wherein the substance producing cell and the non-transformed partner in the n-oma cell line originate from the same specie.

18. A process according to anyone of claims 16 and 17 wherein the substance producing cell and the non-transformed partner in the n-oma cell line are of any animal specie origin, including human.

19. A process according to anyone of claims 16 to 18 wherein the substance producing cell is an eukaryotic cell.

20. A process according to anyone of claims 16 to 19, wherein the substance produced is any animal protein.

21. A process according to anyone of claims 16 to 19 wherein the substance producing cell is a lymphocyte.

22. A process according to anyone of claims 16 to 21 wherein the substance produced is a monoclonal antibody.

23. A process according to anyone of claims 19 to 21 wherein the substance producing cell is presensitized either in vitro or in vivo to produce the predetermined substance.

24. A continuous, stable, secreting n-oma cell line which is the fusion product between the n-oma cell line according to anyone of claims 10 to 15 and a substance producing cell according to anyone of claims 17 to 19, 21 and 23.

25. A process for producing a monoclonal antibody which comprises culturing the n-oma cell line of claim 24 in an in vitro or in vivo culture medium and thereafter isolating the monoclonal antibody from said culture medium.

26. A process according to claim 25 wherein the in vivo culture takes place in any desired animal which has been previously made permissive to the n-oma cell growth by any genetic or biochemical manipulation.

27. Monoclonal antibody produced by n-oma cell lines according to anyone of claims 24 to 26.